Europäisches Patentamt

European Patent Office

Office européen des brevets

(11) Publication number: **0 327 786**
**A1**

# (12) EUROPEAN PATENT APPLICATION

(21) Application number: 88810081.5

(51) Int. Cl.4: **G01N 33/52 , G01N 33/53**

(22) Date of filing: **11.02.88**

(43) Date of publication of application:
**16.08.89 Bulletin 89/33**

(84) Designated Contracting States:
**AT BE CH DE ES FR GB GR IT LI LU NL SE**

(71) Applicant: **IntraCel Corporation**
**c/o Cottle Catford & Co. 17 High Street**
**Bridgetown(BB)**

(72) Inventor: **Sutherland, Ranald**
**Appartment C3, 535 Hinman Ave.**
**Evanston Illinois 60202(US)**
Inventor: **Hybl, Eva**
**4, Av. Frédéric-Soret**
**CH-1203 Genève(CH)**
Inventor: **Bregnard, André**
**35, avenue du Lignon**
**CH-1219 Le Lignon(CH)**
Inventor: **Place, John**
**42, chemin de la Chevillarde**
**CH-1208 Genève(CH)**

(74) Representative: **Dousse, Blasco et al**
**7, route de Drize**
**CH-1227 Carouge/Genève(CH)**

(54) **Method and apparatus for carrying out chemical or biochemical reactions in porous carrier phases.**

(57) One of the reacting partners whose concentration does not substantially vary during the reaction is immobilized in the porous carrier phase. A solution of the other partners in a liquid is passed through the carrier whereby the reaction occurs and generates a signal which is picked up from the exhaust liquid. The magnitude of the signal depends on the rate of flow of the liquid through the carrier. This rate is kept under control and constitutes one of the parameters useful in measuring said reaction.

FIG. la

FIG. lb

EP 0 327 786 A1

# METHOD AND APPARATUS FOR CARRYING OUT CHEMICAL OR BIOCHEMICAL REACTIONS IN POROUS CARRIER PHASES

## DOMAIN OF THE INVENTION

The present invention concerns a method and an apparatus for carrying out chemical reactions in which one or more reaction partners are attached to a solid carrier. Such reactions are particularly useful for the determination of chemical or biochemical species in analytical samples of solutions or biological fluids, these species being either directly reacted with reactants bound or absorbed on carrier surfaces, or being themselves first immobilized on said carriers and thereafter reacted with reactants specific thereto in a signal generating reaction.

The method and apparatus of the present invention are particularly useful in the field of medical and clinical diagnosis, with special reference to immunotests and nucleic acid probe tests, although essentially not limited thereto.

## THE PRIOR ART

Within the science of in-vitro diagnostics, some of the most useful tests are those based on immunodiagnostics. These tests are clinically sensitive and clinically specific, and are thus very important in terms of patient diagnosis and treatment. Examples are in monitoring drug therapy, monitoring diabetes treatment, detecting pregnancy, detecting and monitoring various cancers, detecting microbial infections, detecting and monitoring disfunction in lipid and lipoprotein metabolism, and in the detection of viral infections such as hepatitis or the HIV 1 family.

Immunodiagnostic assays are based on the reaction of a specific binding entity, the antibody, with its binding partner, the antigen, in a tight, rapid and effectively irreversible reaction sequence. The antibody, a glycoprotein molecule, generally of the immunoglobulin G (IgG) class, with a molecular weight of approximately 150,000 Daltons, has two binding sites in its natural form. Each binding site specifically recognizes a certain (normally the same) 3-dimensional structure (the epitope) on a target antigen molecule. The antibody is very specific against this epitope and ideally binds tightly and effectively irreversibly with a very high affinity to the antigen. In this fashion, biochemical analyses based on antibody-antigen reactions (i.e. immunoassays) are very specific due to the specificity of the reaction, and sensitive due inter alia to the affinity (i.e. tightness of binding) of the reaction.

Due to this specificity and sensitivity, antibody-antigen reactions (i.e. immunoassays) are extremely useful in many areas of analytical biochemistry, especially clinical medicine, as in diagnostic/prognostic medicine. Generally the aim is to detect and/or monitor compounds that indicate a disease state which exists in a very low concentration ($<\mu$mol/L) in the body fluids, in the presence of a factorially higher concentration of physically similar compounds. Detection of such compounds (the target antigens) is normally achieved by obtaining the appropriate high affinity antibodies by conventional means and designing an assay system to allow the sensitive, specific detection of the antigens. In a simple form, i.e competitive immunoassay, such a system involves using a known, defined concentration of purified target antigen which carries a label (which can be identified thereafter by a variety of techniques), and a known, defined concentration of specific antibody. The target antigen and the labelled antigen compete in binding with the specific antibody, so that the target antigen and the analytical sample reduces the proportion of labelled antigen bound to the antibody. This gives the basis of a dose-response relationship whereby a sample with an unknown concentration of target antigen can be subjected to this basic procedure and an accurate estimate of target antigen concentration can be obtained by simply running the "unknown" sample versus known standard target antigen solutions, and interpolating the data.

One of the key requirements in running such immunoassay systems is to distinguish the measurement signal from the free species from that given by complex bound label. This is usually achieved by physically separating the free and the bound species prior to detection of specific bound or free signal.

The means of carrying out such separations are extensive. In general, this "separation" step is one of the most analytically critical steps in an immunoassay test sequence being the largest source of errors. To circumvent this pitfall, systems in which it is possible to distinguish the signal of the free label from that of the bound species without effecting a material separation, i.e. without a manipulative step, have been proposed. However, these techniques defined as "homogeneous" immunoassays) have been moderately successful in analytical terms.

2

Indeed, none of these non-separation systems have a high sensitivity and are limited either to high molecular weight, or to low molecular weight target antigens due to the basic physico-chemical nature of these systems. Thus, achieving high sensitivity assays of utility over a broad range of target antigen molecular weights generally requires an immunoassay with a separation step, i.e. a heterogeneous immunoassay.

Now, as mentioned above one of the most important driving forces in modern diagnostic medicine is to carry out highly sensitive and specific assays in a technically unsophisticated testing environment (e.g. doctors' office). However, as was also mentioned, to meet the appropriate analytical criteria requires the use of a separation step in the assay protocol, which makes the assays most complex and technically demanding. Thus within the industry and universities, research has been directed at carrying out separation immunoassays in a simple format which requires minimal operator involvement. It is one aim of the present invention to meet this need. Also, in specialist laboratories with large sample and test workloads, there is still a need for a reliable fully automated system to carry out such immunoassays. It is a further aim of the current invention to also meet this need by supplying the basic component of such a system.

Developments of such sample separation immunoassay techniques are usually based either around a unique physical format which requires no instrumentation and minimal operator manipulations (e.g. accurate pipetting, etc.) or is directed at automating the major steps involved in an assay sequence using specially designed instrumentation which effectively automates all the manual procedures in an assay sequence. Although the latter is an expensive and complex solution, accurate, reproducible and quantitative results are frequently not achievable by other means. Currently a small, highly sensitive, specific and quantitative analyzer for carrying out such biochemical and chemical analyses as mentioned above does not exist. This invention relates to a new type of assay format, especially useful to immunoassays, which allows for the first time the development of both a quantitative or qualitative assay systems for not only the decentralized testing environment but also the central laboratory.

Basically, the technique used here involves a carrier phase to which reagents are immobilized by absorption or by bonding. In the simplest case, one of the reaction partners of an immunopair is immobilized on the carrier and the other partner, usually in solution, is contacted with the carrier, whereby immunocomplexing occurs. One of the reagent partners carries a marker capable of generating a signal to be subsequently measured. Usually this marker may be a radioactive isotope, a fluorescent or coloured label or an enzyme signal generator. Then, following the reaction, the excess solution is washed away from the carrier phase to separate the unbound label from bound one and the signal is measured, depending on reagents used and immunoassay types, either on the carrier, or in the solution, or both.

Relevant prior art in this area includes the use of different types of solid phases to support and be used to separate antibody-antigen reaction products at appropriate times. There are many types of solid phase which have been used. Some examples are the surfaces of plastic tubes or wells, and of plastic balls or beads (refs. K.J. CATT, G.W. TREGEAR et al, Clin. Chim. Acta (1970), 27, 267-279; E. ENGVALL and P. PERLMANN, J. immunol. (1974), 109, 129-135). Also, there are many microparticulate solid phases such as microparticulate cellulose (ref. L. Wide et al., Biochem. Biophys. Acta (1966), 130, 257), controlled pore glass particles (ref. H.H.Weetall et al., Biotechn. Bioengin. (1973), 15, 455, CORNING GLASS WORKS), magnetizable microparticulates and microfine cross-linked dextran beads (see also US patent No 4,425,438). However, with coated macro-surfaces such as beads or the interior of tubes, the surface area for reaction is very limited thus limiting the maximum specific signal. Also, as the majority of the target antigen is physically distant from the reaction surface such systems are effectively diffusion-limited. On the other hand, with the microparticles the effective surface area is extensive allowing high concentrations of specific reagent to be introduced into the assay and also reducing the diffusion limiting factors found with the "macro" solid phases. However, the microparticulate solid phases are difficult to handle and extremely demanding in terms of physical separation, centrifugation being the most commonly used technique.

What was required was a new type of solid phase which allows all the benefits of fast reaction rates with the benefits of simple separation, e.g. a porous phase carrier. Indeed, microparticulate solid phases are fundamentally unsatisfactory because they represent the dispersion of the solid within the liquid, and mixing and separation are achieved by moving the solid within the liquid. A more satisfactory and practical way of resolving this problem is to disperse the liquid phase within the solid phase and achieve mixing and separation by moving the liquid within the solid. Increased surface area contact is achieved by dispersing the liquid phase. The latter approach has three distinct advantages. First, a dispersed liquid phase is relatively easily gathered into a homogeneous phase (in contrast to a particulate solid phase which cannot be reaggregated). Second, the target antigen itself is dispersed within the liquid phase and by controlling the dispersion, the liquid phase can be brought into contact with the reactive surface of the solid phase. Third, the movement of the liquid phase through a solid phase reactive zone allows for the scavenging of target antigen and increases the efficiency of complete reaction (compare absorption or affinity columns). The porous carrier thus offers distinct advantages over the microparticulate carrier. Such porous carriers are available in the familiar form of filter papers and, more recently, in the form of physically similar but microscopically and chemically different material, i.e. membranes. These membranes are commercially available and have the physical characteristics of rigidity, tensile strength, are unreactive to many chemical solvents and have a regular porous structure. A number of commercial products have also been chemically modified to give a highly active surface to allow the simple rapid immobilization of high concentrations of specific reagent within the membrane structure (e.g. Biodine TM, PALL CORP., Glencove, NY 11542, U.S.A.; Immobilon TM, MILLIPORE; nitrocellulose, BIORAD, Griffin Av., Richmond, CA 94804, U.S.A.). These types of solid-phase retain the positive factors of both the microparticulates and the macro solid phases with only few of the disadvantages.

There are a number of examples in the art which exploit the basic utility of such porous solid-phase support materials for carrying out assays. Most examples relate to the structure of the receptable in which the solid-phase is held, and in what manner the various solutions are passed through the solid-phase. In patent US 3,888,629 it is taught how to perform reactions within an absorbent pad such as a filter. Here a reaction cell is described which comprises a container holding a matrix pad in which the reaction takes place, the container so structured as to allow passage of all liquids through the pad. This technique was designed mainly for use in assays utilizing radioisotopes. The present testing requirements would make such a system somewhat obsolete. A further reference can be cited from the art in patent US 4,246,339, describing a test device with two components or members. These members are so disposed to allow relative movement towards or away from another allowing the membrane in which the specific reagent is carried, to contact an absorbent pad which acts as the wicking agent, pulling solutions through the membrane when physically in intimate contact with the membrane. A further reference is patent US 4,623,461. Here a test device is described where the design allows transfer of liquids through the solid-phase reaction matrix not passing through the filter, but passing transversely, flowing outward from the site of application into an absorbent. The claimed advantage of this reference is to have more accurate results and the possibility of reading the signal from either side of the membrane in which the specific signal is formed. A further example of prior art is described in patent US 4,632,901 whereby the specific reagent is bound to the supporting membrane or filter within the area smaller than the area of sample application. There is also described another embodiment in which an absorbent pad which contains microcapillaries is placed in direct contact with the membrane. Other relevant prior art includes patent US 4,407,943, US 4,366,242 and EP 0,0200381. In most of the prior art, the generated reaction signal, generally a developped colour or fluorescence, is observed directly by reflexion from a porous absorbing phase; therefore the result is qualitative rather than quantitative. For instance, in all the above cases, except the first example, it is envisaged that the reaction product can be detected as a precipiated colour within the solid-phase. Typically one partner of the binding pair may be conjugated to an enzyme. Following the various reactions the bound enzyme is detected by adding a chromogenic substrate which precipitates within the reagent pad structure, and the resultant colour formed can be visualized and may be compared to standard colours. This approach is only adequate for qualitative or semi-quantitative analyses due to the nature of the signal. It is difficult to accurately quantitate a dye which is precipitated within a membrane or filter pad structure. The preference would be to measure the colour formed in a solution within an accurately defined optical cell of known physical dimensions.

However, the problem of avoiding diffusion-controlled reaction conditions in a porous matrix remains. Even when using microparticulate solid carriers where the reaction partners are brought together by vigorous agitation, it is estimated that an unmixed liquid layer some hundreds of $\mu$m thick may remain on the surface of the solid phase making the overall reaction diffusion controlled. Therefore, a detailed knowledge of the intimate structure of the porous carrier medium is very important if one wishes to overcome the diffusion problems inherent to operating heterogeneously; apparently, such problems do not exist when operating under the homogeneous conditions prevalent in the freely available space of a normal analytical cuvette. These problems and how to select appropriate porous reaction carriers have been discussed in detail by R.E. AXEN et al., in EP-A-134.236 (PHARMACIA).

The ideal porous support to effect analytical reactions with reagents immobilized therein provides sufficient surface area to immobilize sufficient reagent in a relatively small volume of material so that the overal space occupied by the reaction site is about equal, or does not much exceed that of an ordinary analytical cell. Furthermore, the porous structure should ideally be such that the reaction rate of the bound reagents with the analyte be at least substantially independent of the diffusion rate of the dissolved analyte within the pores of the matrix.

In addition to allowing good contact between the analyte and the specific reagent, heterogenous microanalytical methods such as immunoassays also require a complete separation of the complex or bound analyte from the analyte free in solution. Poor separation is well known to be the major cause of imprecision in such assays. The ideal support should therefore also allow good separation with maximum convenience. Quantitative assays using microporous matrices most often require repetitive washing steps to separate out the free moiety from that bound within the matrix. To a certain extent the smaller the pore size in a porous matrix (and consequently the better the contact between reactants), the more difficult it may become to separate completely the liquid phase after reaction.

Thus, although a porous matrix may present an aspect of pratical separation convenience to the user, it introduces difficulties with respect to complete contact between analyte and its partner reagent and to complete separation of the bound and free moieties.

In the prior art techniques the primary liquid (usually the sample or a dilution of the sample containing the analyte) is generally added, by means of a pipette or otherwise, to the dry matrix. This liquid is drawn in the matrix and retained therein by capillary forces in the matrix pores. In order to obtain a quantitative determination of the analyte, it is important that all the active surface of the pores is wetted with the liquid and that no air is trapped within the matrix. Usually, wetting agents are added to the liquid or are present within the matrix to ensure complete filling.

When the primary reaction is complete, the liquid is removed from the matrix. This removal can be effected by squeezing to expel the liquid (if the matrix is compressible), by aspiration e.g. under vaccum or by contacting with a porous pad or sponge, the capillary power of which exceeds the capillary retaining ability of the matrix. However, these techniques are not really satisfactory on a quantitative and manipulative basis for several reasons. One reason is that expressing a liquid out of a porous matrix is rarely complete and aspiration with a suction device may introduce undesirable air bubbles into the pores of the matrix which may be difficult to remove again and which tend to trap liquids in a dead volume. Repeated washing and aspiration is often used to save time and completely separate the primary liquid. Such washing and aspiration steps require extra manipulations and addition and disposal of comparatively large volumes of liquid.

Furthermore, in case the primary reaction does not itself generate a measurable signal, one or a number of liquids are added one after the other to complete the reaction and provide the necessary signal.

Hence, the final reagent that is added to provide a measurable signal allowing determination of the analyte is often allowed to remain in the matrix where the colour is observed or the absorbance measured by reflectance. As said before, both of these measurements are subject to errors, either because of subjectivity or because of dispersion or absorption of the light signal by the matrix. A better quantitative measurement would be obtained by removing the coloured end product, e.g. as a solution, and measuring it in a conventional spectrophotometer.

Accurate determinations require that certain liquid additions (the sample and the substrate addition at least) be carried out in a reproducible and quantitative fashion. Quantitative addition and reproducible removal of liquid phases in a porous matrix is therefore necessary to obtain accurate and reproducible results. Effective removal of liquid can be accomplished by washing, but the use of large volumes of liquid does not improve handling convenience. When the analytical reactions involve the use of successive additions of small volumes to a porous matrix, complete separation and avoidance of contamination is particularly difficult.

While substantially "dry" chemistry system have been successfully commercialized for higher concentration analytes (such as the reagent film system of Kodak), using conventional colour reactions, the use of similar systems for microanalytical determinations such as immunoassays, has proven to be much more difficult. In dry chemistry systems the wetting of the porous layer is accomplished using a wettable distribution layer in close proximity to the reagent containing layers. Samples are added in precise volumes and the liquid penetrates the multilayered system to produce finally a coloured product that is measured by reflectance. Since this type of conventional colour reaction does not require a separation step or the successive additions of liquid reagents, the problems of liquid displacement are less demanding than for immunoassay type reactions.

## DESCRIPTION OF INVENTION

Now, the method of the present invention, as summarized in claim 1, offers a route to remedy the foregoing drawbacks associated with the use of porous matrices as reaction site for carrying out analytical reaction.

Briefly, a type of reaction is envisaged in which the kinetics is zero order or pseudo-zero order, i.e. one of the partners is not consumed in the reaction (e.g. a catalyst like an enzyme) or its concentration is large enough relative to the other partner so that its variation during the time the signal is measured becomes negligible. The invariant (or pseudo-invariant) partner is immobilized in the porous matrix and the other partner dissolved in a liquid, is passed through the matrix at a given flow rate (this flow rate should be kept the same for all analyses of this type). Since the reaction takes place at a certain rate determined by the relative instantaneous concentration of both partners, the extent of signal (to be measured as a property change in the liquid exiting from the porous body) is inversely proportional to the flow rate.

Thus, in a first case (A) where the species to be determined is the invariant partner (or a direct precursor thereof) immobilized in the porous body, the test is carried out by passing through the body at a given flow rate a solution of known concentration of the other partner. During reaction, a signal is generated (e.g. a colour develops in the exiting solution) which can be measured at the output of the porous body, for instance by draining the used solution through suitable probing means. The slower the flow rate, the deeper is the observed colour down to a certain lower level of flow where the reaction is complete (i.e. all the other partner is consumed in the reaction). Therefore, operating at flow rates below this lower level is no longer possible within the scope of this invention, the point being that any rate between said lower flow rate level and complete immobility provides the same response; therefore the rate is no longer a useful parameter and very slow rates must be avoided unless additional criteria are considered, e.g. clock factors (see later).

In case (B) where the species to be determined is in solution, the second reaction partner (in large stoichiometric excess) is immobilized in the porous body and the solution is passed therethrough. The same phenomena (as described under A) occur, i.e. the response is in direct relation to the concentration of the species in the same and in inverse relation to the rate of flow. Determinations are made with reference to calibrating standard solutions, using the same rates of flow in all tests.

## DETAILED DESCRIPTION OF THE INVENTION

Variant (A) is exemplified by sandwich type immunoassays. For instance, an analyte species, e.g. an antigen is immobilized in a porous matrix by means of a first antibody bound thereto beforehand. Then, a second antibody labelled with a marker, e.g. an enzyme is added which quantitatively binds to the antigen. The excess of labelled antibody is washed away (incidentally, the washed-away portion can also be measured if desired) and an enzyme substrate of known concentration is passed at a controlled rate through the porous body with the enzyme label, whereby a response is provided in proportion to the amount of immobilized enzyme. This response (for instance a colour, a fluorescence, a turbidity, a refractive index change, a dielectric change, a conductivity change, or any other property) is measured by passing the exiting liquid through an appropriate detector. The response depends on the rate of flow and on the marker activity only, not on the volume of the enzyme substrate if all tests are carried out at the same rate.

In other embodiment to be applied to competitive immunoassays in which an antigen Ag must be measured in a sample, a known amount of labelled Ag* is added to the sample and the mixture is immobilized on a porous carrier by means of an antibody (Ab) attached beforehand thereto. Obviously the amount of Ag* immobilized effectively is inversely proportional to the amount of Ag present. Then the immobilized Ag* is determined by the same technique disclosed above, e.g. by passing a solution of enzyme substrate through the carrier at a controlled rate and compared with results obtained from Ag standards run in the presence of the same amount of Ag*.

Conversely, the test also applies to excess reagent assays. In this case a known excess of labelled antibody Ab* is reacted with a sample and the mixture is passed through the carrier which contains, immobilized, an antigen. The uncomplexed Ab* then attaches to the carrier and is thereafter determined as above.

Nucleic acid sequences can also be determined according to the present invention. For this, a sequence complementary to the sequence of interest is attached to the porous carrier and the sample is run therethrough afterwards, whereby the sequence of interest is bound by hybridation. The duplex is then identified with a nucleic acid probe carrying a signal generating label, using the foregoing technique. This is the nucleic acid and probe test equivalent of the sandwich assay. Nucleic acid tests can also be carried out using equivalents of competitive and excess reagent assays.

An example of variant B is provided by attaching an excess of enzyme substrate to a porous carrier body and passing therethrough a solution of an enzyme to be measured. The product of the reaction of the enzyme with its substrate (colored product) is drained off the porous carrier and measured as said before under controlled rate conditions. Obviously, since the amount of enzyme substrate remains substantially constant during the test, the response for a series of experiments run at the same rate program depends essentially on the concentration of the enzyme in the sample, not on the volume of the sample.

Actually, the rates in this invention can be constant or variable during a test and the rates controlled actively or passively. Active rate control can be provided by usual means known in the art, i.e. controlled pumping or aspirating means.

Tests in which rate varies and passive rate control is exercized, relate to cases where the liquid flows through the porous matrix by gravity i.e. the liquid flows under its own weight. This is a particularly interesting case because it is very simple to perform and because although the rate of flow varies with the height of the liquid column it remains non-dependent of the volume of liquid used. More specifically, in this case the porous body (which can be a filter, a pad, a layer of fibers, a porous membrane, or any layer of inert porous material) is placed at the bottom of an upper channel or vessel and the liquid (with a known fixed concentration of reagent) is poured over it. The liquid flows through the porous material by its own weight and if this material is correctly wettable by the liquid, the last portion will be retained in the pores by the capillary forces and the latter portion thereof will fill a compartment (e.g. a drain channel) under the porous body because of the surface tension in the liquid (of course the diameter of the drain channel below the porous body should be small enough not to let the air enter from below). This way, the measurement of the signal in the exiting liquid can be made when the liquid stops flowing, e.g. at a given fixed distance below the porous pad, thus ensuring very accurate measurement reproducibility although independent of the volumes of liquid added to the upper channel. Therefore, accurate pipetting is unnecessary. Note that the signal is generated before the flow stops; only the measurement of the signal is performed after the flow has stopped. This will be explained in detail hereafter.

A device for embodying the present method will now be described with reference to the annexed drawing.

Fig. 1 is a schematic vertical cross-section of a device for carrying out the method of the invention. Fig. 1a is the upper vessel part of this device which fits snugly (water tight seal) into the opening of the lower part schematized in Fig. 1b.

Fig. 2 is a cross-section of the device of Fig. 1 along line II-II.

Fig. 3 is another cross-section along line III-III of Fig. 1 showing schematically a light source and a light detector.

Fig. 4 in an enlarged and diagrammatic vertical cross-sectional view of a portion of the device.

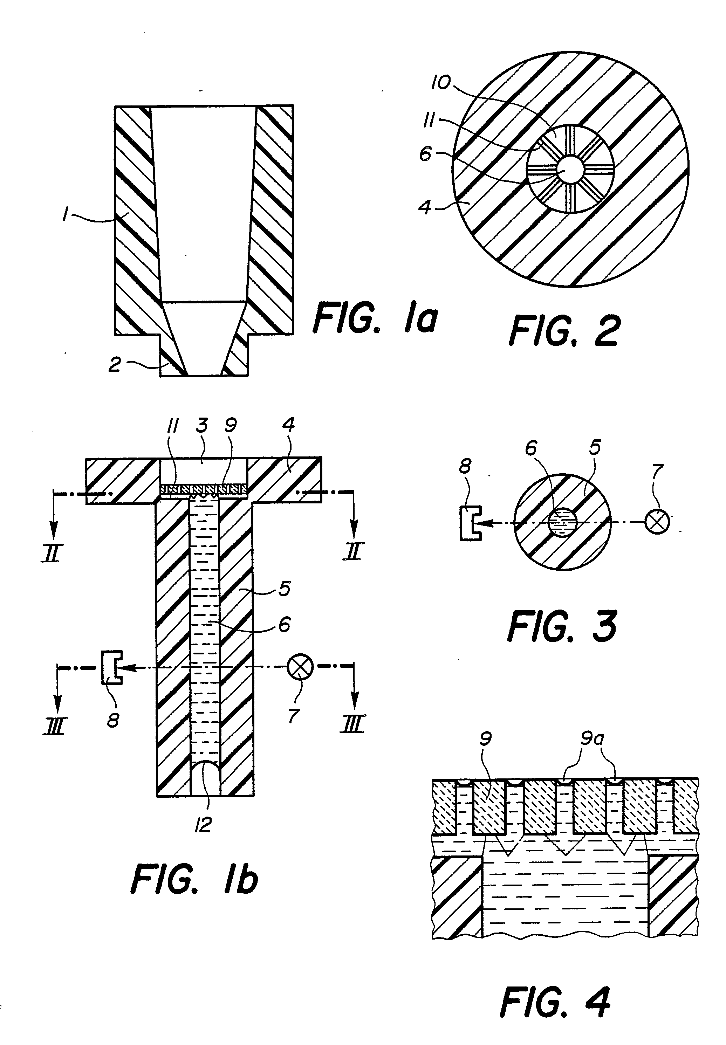

The device represented schematically in Fig. 1, comprises a (cylindrical) upper vessel 1 made of plastic (Lucite, PMMA, PVC or the like) and provided with a portion 2 of reduced diameter. This portion 2 seals in a wall 3 of a lower portion 4 (see Fig. 1b), also made of plastic but detachable at will. The bottom of well 3 opens into a discharge tube 5 of which the hollow internal duct 6 is radially crossed by a beam of light from a source 7 (see Fig. 3). After crossing tube 5, the beam of light is picked up by a light detector 8.

A porous body 9 with pores 9a is placed at the bottom 10 of well 3. This bottom 10 is provided with (small) grooves 11 which open into the central duct 6 of tube 5.

A typical sandwich immunoassay in which a first antibody is immobilized on the porous matrix and a second antibody labelled with an enzyme is added to complete the sandwich containing the analyte is given to illustrate the operation of the foregoing device. Thus, the present device is operated as follows:

First a reagent specific to an analyte to be detected is immobilized onto the porous membrane body 9 which can be made of any porous material suitable for immobilizing reactants in the pores (i.e. plastics, glass, porous silica, foam, etc.). Then the porous body 9 is placed at the bottom 10 of the well 3 and the reduced size portion 2 of upper vessel 1 is fitted into the well 3.

The sample containing the analyte to be determined (in this case an antigen) is added to vessel 1 wherefrom it enters into the porous body 9 in which a reaction occurs between the antigen and the specific antibody immobilized in the matrix pores. Liquid in a volume exceeding that of the matrix pore volume collects downside in the inside of tube 5. This liquid can slowly fill the exit channel depending on its volume and at a rate determined, namely, by the porosity of body 9, the characteristics of the liquid (viscosity, surface tension) and the hydrostatic pressure of the liquid column. It should be noted at this stage of the test that no signal is generated, therefore the rate at which the antigen solution crosses the matrix has no analytical significance yet. It should however be sufficiently slow to ensure quantitative immobilization. Hence, the microporous matrix acts here like an affinity column and liquids passing through the matrix are fully reacted by the time the liquid reaches the lower part of the individual pores.

Once all the liquid has passed into the porous body 9, i.e. when the top level of the liquid reaches the upper level of the pores 9a in the porous body, the downward flow stops because some further downward displacement would work against the capillary retaining forces in the pores 9a which tend to keep the pores filled with the liquid. This is because the matrix is wettable and the contact angle between the liquid and the

7

matrix will provide an upward capillary force that resists further downward movement of the liquid.

At the same time, a meniscus 12 forms at the liquid bottom end in the discharge duct 6. At channel diameters less than 5-6 mm, this meniscus prevents the tube from self-emptying and from allowing air bubbles from below to enter the tube and the space between the underside of the body 9 and the bottom 10 of well 3. It should be noted in this regard that the void space below said porous body, i.e. the grooves 11, is shaped so as to ensure that all air which may have been trapped originally in this space is entrained with the flowing liquid.

In the collecting channel which preferably has a wettable surface (although non-wettable surfaces are acceptable if the channel dimensions are small) the liquid forms a meniscus that tends to draw the liquid downwards. Below a duct diameter of some 5-6 mm this meniscus tends to resist the possibility of air bubbles forming in the tube. If the liquid reaches the end of the outlet tube, the meniscus becomes reversed thus resisting further downward movement of liquid. Since the meniscus is now convex with respect to the outlet tube, it is relatively easy to contact and break by applying a wettabe absorbing pad.

Some care needs to be exercized in contacting the bottom end of the discharge channel wich such an absorbing pad. If the pad remains in contact with the channel walls, liquids are able to move into the pad and will eventually empty the channel. This can be avoided by placing the absorbing pad at a small fixed distance (e.g. 0.1 to 5 mm) from the bottom of the discharge channel.

If a non-wettable surface is used for the exit channel, the absorbing pad has less possibility to empty the channel. In this case, the exit channel must be of smaller "capillary" dimensions to allow the flow of liquid to displace any air originally present below the matrix.

The cross-sectional dimensions of the channel and the properties of the material of the internal walls of this channel are thus of importance in forming the meniscus and preventing air bubbles from penetrating into the liquid and moving upwards by buoyancy. Normally, a cylindrical collecting duct (or other geometrical form of practical equivalent capacity) not exceeding about 5-6 mm diameter is suitable when made of extruded plastic like PVC, polystyrene, PMMA and the like, or glass. However this tube can also be a capillary tube of much smaller cross-sectional size (e.g. 100 - 500 $\mu$m) which better suits embodiments involving very small reaction vessels, e.g. three mm wide cuvettes and smaller volumes.

A second liquid containing the enzyme-labelled second antibody to the analyte is then added to the vessel 1.

The capillary retaining forces at the surface of the matrix vanish and the new fraction penetrates into the porous body and, from there, into the discharge tube, thus displacing the first portion which may then be expelled out of the discharge tube through the orifice thereof. This technique allows the displacement of one liquid portion by another portion with minimal liquid mixing at the interphase which is one of the desired conditions for achieving quantitative measurements.

If the downward hydrostatic force resulting from addition of new liquid and the cancelling of the capillary forces at the matrix surface is not sufficient to overcome the resistance of the meniscus at the outlet of the discharge tube (this could be the case for instance when the tube is of capillary size), this can be again overcome by an absorbing pad or sponge applied in contact with said outlet that disrupts the meniscus at the outlet of the drain channel and takes up the excess liquid.

The microporous body also effectively shields the lower liquids from any admixture with the liquid being added subsequently to the vessel 1. The (small) internal dimensions of the exit channel also allow displacement by successive liquids added with a minimum of admixture at the interface.

One or more rinsing solutions may be added at this stage to ensure separation of non-reacted second antibody.

Finally, a liquid is added that contains the signal generating substrate to the enzyme of the second antibody. As the substrate passes the enzyme immobilized by reaction with the antigen already bound to the matrix, it is converted into a coloured product which then passes into the lower exit channel for optical measurements. The rate at which this liquid passes through the matrix is dependent, inter-alia, on the shape of the upper vessel and the lower channel as well as on the physical properties of the liquid and the porosity of the porous body. It should be noted that, if desired, controllable means, e.g. valve means, can be used in the present apparatus to control the flow of liquid in the channel. In the prior art, this substrate solution must normally be added in known volume for reproducible results to be obtained. Since the method described here relies on measurement of a defined part of the liquid column which has flowed through the matrix, the variations in the volume of the substrate added have little effect on the measurements which are obtained, for instance by measuring at a fixed distance below the matrix when the flow stops.

Each liquid movement may last from a few seconds to about 2-3 min. depending on the size of the device and the nature of the porous matrix, etc.

The reaction product in the liquid has properties which are detected and measured by passing a light beam from the source 7 across the center 6 of the tube 5 and collecting the output signal with detector 8. The signal from detector 8 (a phototube or any other appliance capable of converting light into an electric signal) is processed by usual means (amplifier, discriminator, comparator, display, recorder, etc.) to provide the data required in the measurement.

Thus, the monitoring or measuring steps applied to the liquid in the channel connected to the exhaust portion of the body of porous material are carried out by optical means in which the shape of the channel is important. For instance this channel may be provided with sections in which an incident exiting light beam directed thereto can interact with the liquid and, as a consequence, generates a response signal indicative of the desired determination. Optical interactions between the incident beam and the liquid can involve light absorption, scattering or emission of fluorescence. Thus, the channel can be made of a transparent material, e.g. an extrusion moulded plastic whose shape is adapted to facilitate said interaction of the beam and the liquid. For instance, the channel can be provided with lens shaped portions which will focus the input light beam into the liquid and concentrate the exiting beam on a suitable photodetector. Alternatively, the channel can be shaped so as to behave as a transmission waveguide in which the light travels by multiple total internal reflection, the result being the interaction of the evanescent wave component thereof with the liquid in the channel. Devices representative of these embodiments will be detailed hereafter.

Alternatively, or in addition, means different from optical ones could also be applied in the process, i.e. flow-rate measuring means, conductivity, EMF or dielectric measuring means; or other characteristic feature measuring means.

Many variants to the aforementioned device are possible. For instance, the porous body 9 can be composed of more than one layer in each of which a different reaction may take place e.g. the analyte sample may contain different species to be analyzed each of which will react in one of said layers. In this case the exiting liquids could comprise successive fractions: each of which has a property related to one of said species.

It has been noted before that when a liquid containing a given concentration of a second reaction partner is passed through a porous body containing, immobilized therein, a first reaction partner to be determined, the reaction of the two partners giving a signal which is measured at the output of said porous body, the magnitude of the signal is inversely proportional to the rate of flow of said liquid. If this flow rate is progressively reduced, a plateau of the signal is ultimately reached indicating that the reaction is complete and that no signal change can be expected from that point to complete liquid flow stop. Actually, the position of this plateau (in terms of time) relative to some reference time in the test (for instance a clock mark or flow stop which can be easily determined by checking the level of liquid in the upper vessel) can be used for the desired determination independently of the actual height of this plateau.

The following specific examples illustrate the invention.

## EXPERIMENTAL

## 1. Materials and methods

In general, the porous matrices or bodies used were commercially available. For instance Pall-Biodyne-(TM) immunoaffinity membranes were obtained from Pall Ultrafine Filtration Corporation, NY 11542. These membranes are polymeric, microporous and pre-activated chemically to allow rapid, permanent covalent linkages with high proteins uptake to the membranes Filters papers were generally of the nitrocellulose type obtained from Whatman Limited (Maidstone, England) with pore sizes of 0.45 $\mu$m. With the filters it is well-known in the art that proteins can be made to tightly by non-covalently bind to the filters. Among the reagents, purified antigen, Low Density Lipoprotein (LDL), was obtained by CsCl density gradient ultracentrifugation using standard methods with fresh human plasma as the starting material. Mouse anti-human apoprotein B100 monoclonal antibodies were obtained from Cambridge Medical Diagnostics (U.K.) Ltd., Bournemouth, England; polyclonal rabbit anti-$\beta$ lipoprotein, purified IgG, was obtained from Dakopatts, Copenhagen, Denmark. Enzyme-labelled rabbit anti-mouse reagents were specially selected for low non-specific binding and high specific binding. A Horse Radish Peroxidase (HRP) labelled rabbit anti-mouse Igg was obtained from Pel-Freez, Rogers, Ak, USA and an alkaline phosphate rabbit anti-mouse IgG was from Biorad. Labs, Richmond, Ca, USA. All other chemicals used in the buffers were reagent or Analar grade from Fluka AG or Sigma Chemical Co. Bovine serum albumin was from Sigma and liophilized, low fat milk (Sanolait) was bought from the Swiss Cooperative Co.

9

## 2. Immobilization of antigen or antibody to membrane

In general, with both the nitrocellular filters and the membranes, the agent to be immobilized was diluted in phosphate buffer, 0.1 mol/l, pH 7.5 at a protein concentration of between 100 $\mu$g/ml to 2 mg/ml. The solution was either passed through the membrane in a volume from 5 to 50 $\mu$l, or the whole membrane was immersed in a solution of antibody or antigen, or the antibody or antigen solution was applied to a piece of membrane cut to the appropriate size already placed at bottom of well 3. The solution was incubated with the solid-phase for 5 to 30 minutes at ambient temperature, the solid-phase washed repeatedly (at least 5 times the volume of reagent) then the remaining binding sites blocked by incubation with 0.5% (dry W/V) low fat milk with same buffer for 10 top 60 minutes. The exposed surface area of immobilized reagent was the same as that of sample; however, in variants it is possible to change the surface area ratio of reagent to sample to less or more than one by, for example, adding the reagent to the membrane using a mask which limits the area of reagent affixed to the surface. In this case the mask carries an appropriate pattern (e.g. circle, square, rectangle, diamond, letter, figure, etc.) of the appropriate surface area. In this variant, there will always be some signal remaining in the membrane which allows a visual confirmation of, for example, correct reagent addition, while retaining the ability for accurate precise measurement with the transmission photometric measurement in the exit channel. In an embodiment using two or more tubes run in parallel, i.e. with a duplex system, one test can be a sample and the second a control to see if reagents were added correctly. This can be extended to more than two tests and more than one analyte.

Methods of depositing the reagent to be affixed include automated pipetting techniques, screen printing techniques, and other printing techniques such as commonly used in the printing industry, such as ink-jet printing and electrostatic adherence (e.g. the Xerox process). These techniques allow the rapid, large-scale production with reliable precision and accuracy, necessary, to make a quality product available at low cost.

Following the addition of the specific reagent, the complete membrane surface which will be involved in the reagent and sample addition sequences is treated to minimize nonspecific binding events and keep background signal to a minimum. Many techniques are available to do this, the simplest used herein being a pre-emptive blocking of all such remaining binding sites using an appropriate concentration of a blocking agent. High molecular weight blocking agent include proteins and protein mixtures, and lower molecular weight agents include polyamines and amino-acids (e.g. glycine). Here both bovine serum albumin at a concentration of 0.1 to 5.0% (w/v) and preferably powdered low fat milk at a concentration of 0.1 to 10.0% (w/v) were used. The low fat milk contains amongst other components casein which is known to be an effective reagent used to minimize non-specific binding in membranes/filters. For example, the porous phases were immersed in 0.5% (w/v) powdered milk in Tris/HCL buffer for 15 to 60 minutes with agitation. In the case of the membrane this solution was pre-filtered through a 0.22 $\mu$l filter (AMICON). Following this treatment and air drying, the reagent pads were considered ready for use. If necessary, where indicated, the reagent pads can also be treated using non-ionic neutral detergents or silanizing reagents. The former (e.g. Tween 20 or Brij 36) to increase the hydrophibicity of the solid-phase and the latter (e.g. using alkoxylated substituted alkyl-silanes) to modify the wettability of the solid-phase, depending on requirements.

## 3. Fabrication of the apparatus used in the invention

The experiments outlined below were carried out in a device as described in Figure 1 comprising an upper vessel A (see Fig. 1a) and a lower discharge tube B (see Fig. 1b). The two tubes (A and B) were injection-moulded from crystalline PMMA (DIAKON GF250, from ICI Polymer Division, England) using standard injection moulding equipment and a brass mould. During experiments the design parameters of the two tubes were investigated and the following preferred values were found to suit tube A; internal diameter (range 2-10 mm) length (range 5-30 mm), angle of contact at outlet (15-35°), contact/sample application outlet (0,5-4 mm). The actual devices used for the following experiments had the following specifications tube A; internal diameter 3.75 mm, external diameter 6.00 mm, internal length 8.00 mm, external length 9.40 mm, angle of contact at outlet 20°, sample/contact outlet diameter 1.20 mm; tube B, internal diameter 1.00 mm, external diameter 3.30 mm, internal length 10.15 mm, external length 11.50 mm. With this design 3.00 mm diameter reagent pad discs could be used and it was found that a snap-fit design of junction between the two tubes was sufficiently water-tight for all subsequent experiments.

To assemble the devices the reagent pad of the desired dimensions is cut or punched from a layer piece of pad which, in some cases, may be pre-treated with the reagent. The pad is simply placed onto the correct part of the lower tube B, and the correct end of the upper tube A pushed down until the two tubes are tightly mated. A further advantage of this type of design is that it is nearly impossible to contaminate or damage the reagent pad once it is inserted into the device. For these experiments series of 20 to 100 items were assembled each time. However, for field uses much larger series can be assembled in a minimum of time, i.e. 1,000 or more.

The devices were held in a special support which, for the purpose of these experiments permitted, when desired, an intimate contact of the lower end of tube B with an absorbent material. Varying the absorbent material gave different rates of flow in tube B and the choice of absorbent was taken as a function of desired flow rates and sensitivity of the system.

## 4. Assay experimental design

In experiments to be disclosed below, the immunoassay of Low Density Lipoprotein was used as a model system. This is however only one possible embodiment between many since the present device is conceived to be useful in many immunoassay formats, for many different antigens and for non-immunoassay analyses which may, for example involve immobilized enzymes, enzyme substrates, biological particles or pieces of poly-nucleic acids either single stranded duplex or multiple chained.

The chemistry of immunoassays is well documented regarding quantitative and qualitative measurement of analytes in biological samples. The basic techniques will therefore not be detailed herein but appropriate references to the principles of immunoassay are in R.P. Ekins, "Radioimmunoassay and Related Procedures in Medicine", Proc, Symp. Berlin (West) 1977, I, I.A.E.A., Vienna, 1978, and for Enzyme-Labelled Immunoassays in particular, E. Engvall, Methods in Enzymology, Vol. 70, (1980), pp 419-439.

According to a first general example, the antigen (purified LDL) was immobilized to a reagent pad. This solid-phase antigen was then allowed to react with the sample solution which was pre-mixed with specific anti-human Apoprotein B100 mouse monoclonal antibody. The pre-mixed sample plus antibody was either incubated prior to addition to the reagent pad for a predetermined time, or equally mixed together and substantially simultaneously added to the reagent pad. Otherwise, the antibody and sample solutions were added sequentially to the reagent pad with pre-defined incubation times between these additions. Although a monoclonal antibody was used, it is foreseen that a polyclonal antiserum may also be appropriate depending on the test requirements and availability of reagents. Similarly, although the monoclonal antibody was of murine origin, it is possible that other sources of monoclonal antibodies such as mouse/rat fusion partners, or, similarly mouse/bovine fusion partners may be used in the system. Fragments of antibodies or partially purified antiserum could be used. The decision as to what source of reagent, and what reaction sequence is a function of the required assay sensitivity and timing.

The first assay format is based on the principle that the specific antibody will bind to the antigen pre-immobilized to the reagent pad. By prior mixing of the specific antibody with a sample containing antigen in solution the antibody will bind to the sample antigen and thus not be available for binding to the immobilized antigen and can be eluted or washed through the reagent pad following the reaction. Therefore, samples with a low antigen concentration will leave a relatively higher amount of antibody bound to the pad, while samples with high antigen concentration will leave a relatively lower amount of specific antibody bound to the reagent pad. This type of assay has been called a "competitive" binding assay as the antibody, which is in a concentration lower than both the antigen concentrations, effectively competes in its binding to the immobilized and solution antigens. Detection of immobilized antibody can be by many methods by prior labelling of the antibody using signal generators such as enzymes or fluorescent tags. In the case of these examples however, the bound antibody is detected by incubating the reagent pad, following the specific binding reaction between the monoclonal anti-Apo B100 and the immobilized and solution phase LDC, with an anti-mouse IgG conjugated to an enzyme. This anti-mouse IgG binds to the immobilized mouse IgG and following washing steps to remove the unbound materials, an enzyme substrate is added which generates a detectable signal in proportion to the concentration of immobilized specific mouse monoclonal antibody. Although this first assay system is operated with the antigen immobilized to the reagent pad, the system could equally be operated with the antibody immobilized to the reagent pad and the antigen is then the labelled binding partner. This is particularly useful for small antigens.

The second system used was where a first antibody is pre-immobilized to the reagent pad, the sample solution is added containing antigen which is then bound by the immobilized antibody. A second antibody is then added to the reagent pad which binds to an alternative site (or epitope) on the antigen, thus forming a "sandwich" of antibody, antigen, antibody. Following washing steps to remove unbound materials the second bound antibody can be detected as above. In this case, unlike the first system, the signal is proportional to antigen concentration. This type of "sandwich" assay is also known as a 2-site or excess reagent assay, whereby the relative concentrations of specific antibody should be higher than that of the antigen in the sample. Although the example given of this second system is of sequential addition of sample and reagent, it is equally considered that the second antibody could be pre-incubated for a defined time with the antigen, or both the second antibody and sample antigen added substantially simultaneously to the reagent pad. In the examples detailed in this application, the first pre-immobilized antibody originates from a polyclonal antiserum and the second, alternative antibody is a mouse monoclonal. This is not considered limiting to the invention as either or both antibodies used may be polyclonal or monoclonal derived. Similarly, the antibodies may be mixtures of more than one of different monoclonal or polyclonal derived materials. The choice of reagents in both these cases is a function of the desired assay performance. It is generally well understood that the best performing assays have antibodies which bind tightly to the antigen.

The signal detection system used as an example herein was by using anti-mouse IgG antibodies pre-labelled with an enzyme. Two enzymes were chosen for the examples, one which gave a substantially soluble coloured product (Horse Radish Peroxidase, HRP) and the second which gave a substantially insoluble product (Alkaline Phosphatase, AP) (with the appropriate substrate solutions and incubation conditions). These enzymes, the enzyme substrate reactions, and the characteristics of the products are state-of-the-art and well documented, e.g. in Methods of Enzymology Vol 70 (1980).

### 5. Limited reagent assay protocol

All reactions were carried out at ambient temperature but this is in no means limiting the invention as increased or lowered temperature may be used with this system. The assay buffer was always 0.1 mol/L, phosphate, pH 7.4, with 0.9% (w/v) NaCl, 0.5% (w/v) dried powdered milk and 0.01% (v/v) Tween 20. To air-dried reaction pads held within the devices, and with the antigen (LDL) pre-affixed to the membrane, was added 100 $\mu$l of the pre-mixed solution containing 50 $\mu$l of specific anti-Apo B antibody at a concentration of 1 $\mu$g/ml, and 50 $\mu$l of sample. The pre-mixing step in this case was only 10 seconds. The solution was left to react with the pad for 5 minutes then the pad washed with 2 portions ~ 500 $\mu$l of the assay buffer. The anti-mouse IgG (enzyme labelled) was then added in 100 $\mu$l of enzyme buffer, (Tris buffer, 20 mmol/L, pH 7.5 with 50 mmol/L NaCl and 0.05% (w/v) dried powdered milk) and left to incubate for 15 minutes. The unbound materials were then removed by washing the reagent pad with 4 portions (~ 0.5 ml) of assay buffer and the appropriate substrate solution then added to allow colour formation.

The substrate solution was passed through the porous reagent membrane either by gravity along or at enhanced rate by applying the absorbent pad at the outlet of the discharge channel. However, control and experiments in the same series were always carried out identically to ensure full reproducibility. As absorbent, slightly moistened cotton wool or acrylate forms were satisfactory.

### 6. Excess reagent of "Sandwich" assay

In this case, the rabbit anti-$\beta$-lipoprotein (DAKO) was pre-immobilized onto the reagent pads as detailed above, and the reagent pads left to air dry, within the devices, prior to use. 100 $\mu$l of sample was added to the reagent pad and left to incubate for 10 minutes. The pad was then washed with 2 x about 500 $\mu$l of assay buffer.

The alternative antibody, monoclonal anti-Apo B100 was then added in 100 $\mu$l at a dilution of 1/3,000 in assay buffer and left to incubate for 10 minutes. All unbound materials were removed by washing the pad with assay buffer (~ 0.5 ml) three times. The bound monoclonal antibody was then detected as with the limited reagent assay.

### 7. Signal detection

Two major signal detection methods were used with both a soluble chromogenic and insoluble chromogenic products as comparative controls. The soluble system was using an HRP-labelled antibody and the substrate solution made from o-phenylene diamine/$H_2O_2$ with transmission photometry of the final solution in tube B at λ = 460 nm. The comparative insoluble product system was using alkaline/phosphatase labelled antibodies and the substrate solution of BCIP/NBT (BCIP = 5-bromo-4-chloro-3-indolyl phosphate ρ-toluidine salt; NBT = nitro-blue-tetrazolium chloride).

As outlined above, assays were carried out with standard materials containing different concentrations of LDL. The standards were prepared by taking normal human plasma and submitting it to density gradient ultra-centrifugation. The LDL fraction was removed and all other plasma components re-pooled. To remove the salts used in forming the gradient the LDL-stripped plasma was dialyzed against 10 x volumes of isotonic saline, then the dialyzed material was reconcentrated to is original volume using standard techniques. This base material was used as the zero LDL standard. To construct a series of standards to be used in the assay, the LDL prepared by ultra-centrifugation was concentrated to 2 to 5 g/L (of protein, apo B100) and stored in 5% (w/v) BSA in 0.9% (w/v) NaCl buffered to pH 7.5. This material was added to the LDL-stripped plasma to give standard solutions of LDL varying from 0 to 1 mg/ml of Apo B100. These materials were used in all the assays. The procedure for the first assay system was as outlined above using the soluble chromogen in a standard transmission photometer (UVIKON 680, KONTRON, Switzerland).

Measurements were taken by passing the o-phenylene dia mine/$H_2O_2$ solution through the membrane under its own weight and recording the increasing values of absorbance with decreasing flow rate. The highest value is given below.

| LDL Standard (mg/ml) | 0 | 1.0 |
|---|---|---|
| | (Absorbance at 460 nm ± 0.0002) | |
| Replicate | | |
| 1 | 0.623 | 0.020 |
| 2. | 0.620 | 0.010 |
| 3 | 0.618 | 0.030 |
| 4 | 0.619 | 0.025 |
| X ± S.D. | 0.620 | 0.021 |

These data demonstrate that the transmission photometry system which is oulined in the invention description is feasible and is sensitive. It should be noted that 1 mg/ml of LDL is a normal human serum value.

To correlate the system with the prior art techniques of precipitated chromogen several controls were run as outlined above. Although correlation was obtained, significant results needed much longer incubation time and readings (obtained by reflective measurements) required dismanteling of the tubes and removal of the pads. The advantages of the system of the invention are therefore clear.

8. Excess reagent assay

As described above, the excess reagent assay was initially carried out with the soluble chromogen using the zero and 100 μg/ml standard in triplicate. Again, results are given below on the absorbance readings:

| LDL Standard (μg/ml) | 0 | 100 |
|---|---|---|
| Replicate | | |
| 1 | 0.06 | 0.65 |
| 2 | 0.06 | 0.64 |
| 3 | 0.05 | 0.62 |
| X ± S.D. | 0.057 ± 0.006 | 0.637 ± 0.015 |

Also to again demonstrate the flexibility of the device the precipitated chromogen system was used with a 0 and 1 mg/ml LDL standard. The reagent pads were then recovered and measured by reflexion.

All examples shown are of immunoassays using the measurement of the human apoprotein B (ApoB) component of the Low Density Lipoprotein (LDL) as a model assay. LDL is an interesting analyte as it has recently been strongly and causally associated with coronary heart disease and atherosclerosis. This example is however in no way limiting the use or analytical applicability of the device. Similarly, other affinity reagents such as enzymes, enzyme substrates lectins, other antigens, or strands of nucleic acids could be immobilized to the membrane, and where appropriate the reaction products measured directly.

## Claims

1. A method of determining a chemical or biochemical species in a sample by a signal generating analytical reaction involving at least two reaction partners of which one is said species or a derivative thereof , and in which one of the two partners is immobilized in a porous carrier matrix and the other is dissolved in a liquid which is passed through this carrier matrix whereby said analytical reaction occurs and the consecutively generated signal indicative of said determination is measured in the liquid at an output of said matrix, characterized in that said liquid flows through said carrier at a rate which is controlled, the signal indicative of the desired determination being a function of this rate as well as of the amount of this species in said sample.

2. The method of claim 1, in which said species or its derivative is immobilized in the porous carrier and is labelled with a marker capable of generating said signal whose magnitude also depends on the concentration of the other partner in said liquid.

3. The method of claim 2, in which the signal is measured during the liquid flow at a given time or during a given period relative to the start or finish of flow.

4. The method of claim 2, in which said rate of flow is constant and the signal is measured at any time during the flow of the liquid.

5. The method of claim 2 in which the rate of flow varies in a known and reproducible fashion during the time the liquid is discharged at the output of the matrix.

6. The method of claim 2, in which said signal is measured after said flow stops.

7. The method of claim 2, in which the porous carrier phase is located between an upper channel to which said liquid is added and a lower discharge channel in which the signal is measured in the form of a parameter of said liquid, characterized in that the liquid is allowed to flow downward under its own weight through the porous carrier matrix, its instant rate therethrough being therefore dependent on the height of the liquid in the upper channel and the impedance offered by said carrier matrix and in that the signal measured in the lower channel at a fixed distance from said carrier, this corresponding to a fixed volume in the discharge channel.

8. The method of claim 7, in which said parameter is an optical property like colour, turbidity, optical activity, refractive index, fluorescence, conductivity, EMF or the like which is measured by passing the discharged liquid through appropriate probing means.

9. The method of claim 6, in which the downward going liquid stops flowing when its upper level coincides with that of the porous phase in which it is retained by capillarity forces in the pores, characterized in that further liquid displacement only occurs upon addition of a new portion of liquid in the upper channel, and in that said displacement is effected with minimal contamination at the liquid interface.

10. A liquid channelling device for carrying out the method of claim 1, consisting essentially of an upper channel or vessel to which reagents in solution are added, a porous solid body at the bottom of said upper channel capable of retaining one or more reagents immobilized therein and allowing said liquids to pass therethrough at a constant or variable rate of flow, a lower discharge channel at the output of said porous body for collecting said liquids and discharging them from an orifice, and measuring means about said discharge channel for measuring one or more property of said collected liquids before they are discharged.

11. The device of claim 10, in which the added liquid flows under its own weight and in which a factor controlling its instant rate of flow through the device is the instant level of the liquid therein.

12. The device of claim 10, which further comprises means for controlling the rate of flow of the liquid passing therethrough.

13. The device of claim 12, in which said means are pumping or aspirating means.

14. The device of claim 13, in which said aspirating means consists of a porous pad applied to the orifice of the discharge channel directly against it or at a short distance therefrom.

15. The device of claim 1, in which said measuring means consist of a light beam from a light source applied to the liquid in the discharge channel so that said light interacts with said liquid in said discharge channel to provide a detectable signal, detector means to pick up said signal after said interaction and electonic analyzing means to process said detected signal into data required for said determination.

16. The device of claim 1, in which the discharge channel is of capillary size.

FIG. Ia

FIG. 2

FIG. Ib

FIG. 3

FIG. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 716 121 (T.B. HIRSCHFELD) <br> * Whole document * <br> --- | 1-17 | G 01 N 33/52 <br> G 01 N 33/53 |
| X | WO-A-8 603 589 (PHARMACIA AB) <br> * Page 3, lines 5-19; page 13, lines 27-37; page 14, example; page 18, line 2. - page 19, line 36; figure 2 * <br> --- | 1,2,8-10,12-17 | |
| X | FR-A-2 599 844 (CENTRE NATIONAL DE LA RECHERCHE SCIENTIFIQUE) <br> * Page 3, line 33 - page 4, line 27; page 14, example 3; figure 11 * <br> --- | 1,2,10,12-14,16,17 | |
| X | US-A-4 036 946 (M. KLEINERMAN) <br> * Column 2, lines 13-41; column 2, line 59 - column 5, line 8; column 5, line 65 - column 8, line 11 * <br> --- | 1,2,8-10,12-17 | |
| Y | EP-A-0 253 579 (HYBRITECH INC.) <br> * Column 3, line 7 - column 4, line 5; column 10, line 9 - column 12, line 5 * <br> --- | 1-17 | |
| Y | US-A-4 153 675 (M. KLEINERMAN) <br> * Column 1, line 50 - column 2, line 26; column 2, line 44 - column 6, line 18; column 8, line 51 - column 12, line 23 * <br> --- | 1-17 | TECHNICAL FIELDS SEARCHED (Int. Cl.4) <br><br> G 01 N |
| Y | WO-A-8 202 211 (COMMONWEALTH SERUM LABORATORIES COMMISSION) <br> * Page 5, line 3 - page 6, line 15; page 24, line 1 - page 28, line 23; figure 2 * <br> ----- | 1-17 | |

The present search report has been drawn up for all claims

| Place of search | Date of completion of the search | Examiner |
|---|---|---|
| THE HAGUE | 28-10-1988 | VAN BOHEMEN C.G. |